# EUROPEAN PATENT APPLICATION

(11) **EP 0 763 536 A1**
(43) Date of publication of application: **19.03.1997**
(21) Application number: 96113199.2
(22) Date of filing: 16.08.1996
(51) Int. Cl.: C07D 471/04

(54) **Process for producing 6-aminomethyl-substituted quinoline-benzoic acids**

(30) Priority: 17.08.1995 JP 209776/95
(71) Applicant: ASAHI GLASS COMPANY LTD., Chiyoda-ku, Tokyo (JP); THE GREEN CROSS CORPORATION, Osaka-shi Osaka (JP)
(72) Inventor: Wang, Shuzhong, c/o Asahi Glass Co., Ltd., Yokohama-shi, Kanagawa (JP); Okazoe, Takashi, c/o Asahi Glass Co., Ltd., Yokohama-shi, Kanagawa (JP); Morizawa, Yoshitomi, c/o Asahi Glass Co., Ltd., Yokohama-shi, Kanagawa (JP)
(74) Representative: Hansen, Bernd, Dr. Dipl.-Chem.

(57) **Abstract**

6-Aminomethyl-substituted quinolinebenzoic acids useful as angiotensin II antagonists are easily produced in a short step. A 2-{6-(halomethyl)quinolin-2-yl}benzoic acid (1) is reacted with a secondary amine such as a 3H-imidazo[4,5-b]pyridine (2) in the presence of a base to thereby produce a 6-aminomethyl-substituted quinolinebenzoic acid (4) wherein Z may be a bromine atom, etc., R¹ to R³ each may be an alkyl group, etc. and X and Y may be =N or =CH.

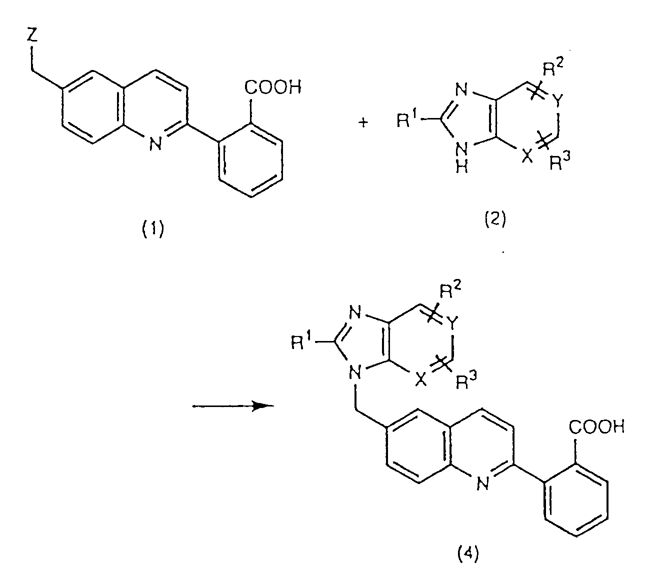

## Description

### FIELD OF THE INVENTION

The present invention relates to a process for producing 6-aminomethyl-substituted quinolinebenzoic acids. More particularly, the present invention relates to a process for producing these compounds which are useful as angiotensin II antagonists and thus are expected to be efficacious as a remedy for hypertension.

### BACKGROUND OF THE INVENTION

As a remedy for hypertension induced by angiotensin II, there have been proposed a number of nonpeptidic angiotensin II antagonists believed to be efficacious when administered orally. Among these compounds, there are known those having a quinoline skeleton such as the compounds described in JP-A-5-230022, JP-A-5-239053, JP-A-6-16659, JP-A-6-80664, etc. (the term "JP-A" as used herein means an "unexamined published Japanese patent application"). Also, there are known processes for producing these compounds, for example, the process described in JP-A-6-16641. Of these quinolines, 2-{6-[(substituted-3H-imidazo[4,5-b]pyridin-3-yl)methyl]quinolin-2-yl}benzoic acids and salts thereof, described in JP-A-6-80664, which relates to an invention completed by the present inventors, are highly excellent in absorption properties *in vivo* and thus are expected to be particularly useful as a remedy for hypertension.

Such quinoline skeleton compounds as those described in the patents cited above are quinoline compounds containing a substituted phenyl group (substituted at the 2-position of the phenyl group), having a group such as -CN or tetrazol-5-yl at the 2-position of the quinoline skeleton, and a substituted aminomethyl group at the 6-position of the quinoline skeleton. Such compounds can mainly be produced by reacting a compound having a halomethyl group at the 6-position of the quinoline skeleton with a secondary amine in the presence of a base. To react such a compound, in which a phenyl group having a -COOH group is bound to the 2-position of the quinoline skeleton, (hereinafter referred to simply as a "2-{6-(halomethyl)quinolin-2-yl}benzoic acid") with a secondary amine, it has been a practice to protect the -COOH group by esterification, then reacting the compound with the secondary amine and deprotecting the reaction product via hydrolysis to thereby give the target compound (see, Examples 1 and 2 in JP-A-6-80664). However, this method suffers from a disadvantage of requiring two steps of protection and deprotection.

### SUMMARY OF THE INVENTION

As the results of extensive studies, the present inventors have found out that a desired 6-aminomethyl-substituted quinolinebenzoic acid can be produced by reacting a 2-{6-(halomethyl)quinolin-2-yl}benzoic acid with a secondary amine, without protecting the -COOH group. Namely, the present invention, the gist of which resides in this finding, is as follows.

That is, the present invention relates to a process for producing a 6-aminomethyl-substituted quinolinebenzoic acid wherein an amine residue which is formed by eliminating the hydrogen atom bound to the nitrogen atom of a secondary amine is bound to the methyl group at the 6-position of the quinoline skeleton, which comprises reacting a 2-{6-(halomethyl)quinolin-2-yl}benzoic acid represented by the following formula (1) with the secondary amine in the presence of a base: 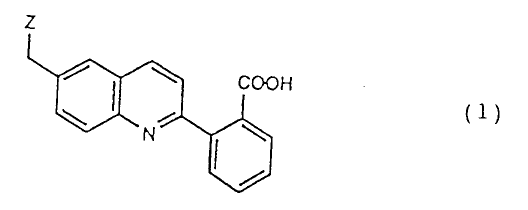 wherein Z represents a chlorine, bromine or iodine atom.

### DETAILED DESCRIPTION OF THE INVENTION

As the secondary amine to be employed in the present invention, various organic compounds having an =NH group can be used. When the hydrogen atom in this =NH group is eliminated together with the halogen atom in a halomethyl group (i.e., eliminated as HZ), a bond, =N-CH₂-, is formed to thereby give a compound having a quinoline skeleton having a substituted aminomethyl group at the 6-position, i.e., a 6-aminomethyl-substituted quinolinebenzoic acid.

Although the secondary amine is not particularly restricted, there are known secondary amines which correspond to the aminomethyl substituents at the 6-position in the quinoline skeleton compounds described in the publications cited above. Examples of the secondary amines include aliphatic secondary amines, alicyclic secondary amines, aromatic secondary amines, and heterocyclic secondary amines. For example, aliphatic secondary amines are exemplified by protected N-valeroylvaline described in JP-A-230022 (corresponding to EP-A-528762). However, it is preferable to use, as the secondary amine, heterocyclic secondary amines corresponding to the substituted amino group described in JP-A-5-239053 (corresponding to EP-A-540500 and U.S. Patent 5,371,227), JP-A-6-16659, JP-A-6-80664 (corresponding to EP-A-569013 and U.S. Patent 5,478,832), etc. The term "heterocyclic secondary amine" as used herein means a heterocyclic compound having a ring containing a nitrogen atom to which a hydrogen atom is bound. Specific examples thereof include heterocyclic secondary amines represented by the following formula (2) having a substituent(s) (in particular, 3H-imidazo[4,5-b]pyridines having a substituent(s)) and 1H-imidazaoles having a substituent(s) represented by the following formula (3):

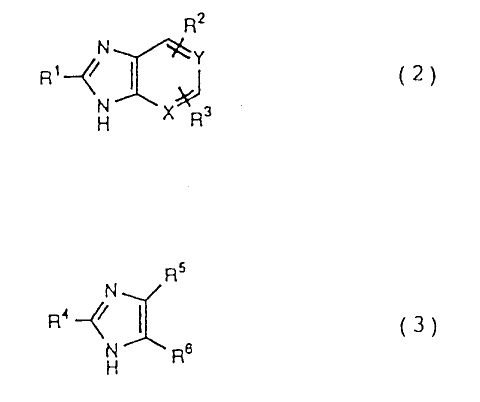

In formulae (2) and (3), R¹ and R⁴ each independently represents a lower alkyl, halo lower alkyl, cyclo lower alkyl, lower alkenyl, lower alkoxy, alkoxy lower alkyl or lower alkylthio group; R², R³, R⁵ and R⁶ each independently represents a hydrogen atom, a halogen atom, a lower alkyl, halo lower alkyl, cyclo lower alkyl, alkenyl or alkoxy group, -CₘF₂ₘ₊₁, -(CH₂)ₙR⁷, -(CH₂)ₚCOR⁸ or -(CH₂)_{q}NR⁹COR¹⁰; and X and Y each independently represents =CH or =N.

The term "lower alkyl group" as used herein means an alkyl group having 1 to 6 carbon atoms. The term "cyclo lower alkyl group" means a cycloalkyl group having 3 to 6 carbon atoms constituting the ring. Similarly, the term "lower" in the "lower alkenyl group", "lower alkoxy group", "lower alkylthio group" and the like means that the group has not more than 6 carbon atoms. R⁷ represents a hydroxyl or lower alkoxy group. R⁸ and R¹⁰ each independently represents a hydrogen atom, a hydroxyl, lower alkyl or lower alkoxy group or -NR¹¹R¹² (wherein R¹¹ and R¹² each independently represents a hydrogen atom or a lower alkyl group). R⁹ represents a hydrogen atom or a lower alkyl group. m is an integer of from 1 to 6. n is an integer of from 1 to 4. p is an integer of from 0 to 4. q is an integer of from 1 to 4.

Examples of the lower alkyl group include methyl, ethyl, propyl, isopropyl, butyl, isobutyl, t-butyl, pentyl and hexyl groups. Examples of the halo lower alkyl group include chloromethyl, 2-chloroethyl, bromomethyl, 2-bromoethyl, 1,2-dichloroethyl, 1,2-dibromoethyl and 4,4,4-trifluorobutyl groups. Examples of the cyclo lower alkyl groups include cycloproypl, cyclobutyl, cyclopentyl and cyclohexyl groups.

Examples of the lower alkenyl group include vinyl, allyl, 1-propenyl, isopropenyl and 1-butenyl groups. Examples of the lower alkoxy group include methoxy, ethoxy, propoxy and butoxy groups. Preferable examples of the alkoxy lower alkyl group are those containing a lower alkoxy group such as 2-methoxyethyl, 2-ethoxyethyl and 3-methoxypropyl groups. Examples of the lower alkylthio group include methylthio, ethylthio, propylthio and butylthio groups. The term "halogen atom" means a fluorine, chlorine, bromine or iodine atom.

Particularly preferable heterocyclic secondary amines are substituted 3H-imidazo[4,5-b]pyridines represented by the following formula (2') wherein the substituent R¹ is a lower alkyl or halo lower alkyl group and R² and R³ each independently is a hydrogen atom or a lower alkyl group. The definitions as to the substituents of formulae (2) and (3) above, apply to the following formula (2'). 2-Ethyl-5,7-dimethyl-3H-imidzao-[4,5-b]pyridine may be cited as the most desirable secondary amine.

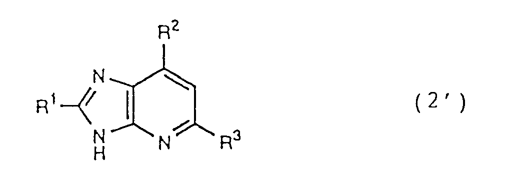

In the compound represented by formula (1), Z represents a chlorine, bromine or iodine atom. A bromine atom is particularly preferable.

The base to be used herein may be appropriately selected from among alkali metal hydrides, alkaline earth metal hydrides, alkali metal hydroxides, alkali metal alkoxides, alkali metal carbonates, and the like. It is particularly preferable to use an alkali metal compound therefor. As the alkali metal, it is preferable to use sodium or potassium. Specific examples of the base include sodium hydride, calcium hydride, sodium hydroxide, sodium methoxide, potassium t-butoxide, sodium carbonate and potassium carbonate.

It is preferable to react the 2-{6-(halomethyl)quinolin-2-yl}benzoic acid with the secondary amine in a solvent. As the solvent, it is possible to use various solvents including aprotic ones. For example, aprotic solvents which can be used include N,N-dimethylformamide, N,N-dimethylacetamide, N-methyl-2-pyrrolidone, tetrahydrofuran and dioxane. Protic solvents which can be used include methanol, ethanol, 2-propanol and 2-methyl-2-propanol. The reaction is preferably carried out at a temperature of from 0°C to the reflux temperature of the solvent. The reaction time is preferably from 0.5 to 10 hours.

The 2-{6-(halomethyl)quinolin-2-yl}benzoic acid employed as the starting material may be produced by the methods described in the publicly known literature as cited above. For example, a halogenating agent and a radical initiator are added to 2-(6-methylquinolin-2-yl)benzoic acid and reacted in a solvent under heating to thereby give a 2-{6-(halomethyl)quinolin-2-yl}benzoic acid. Preferable examples of the halogenating agent include chlorine, bromine and N-bromosuccinimide. Preferable examples of the radical initiator include azobisisobutyronitrile and dibenzoyl peroxide. As the solvent, it is preferable to use a halogenated hydrocarbon solvent such as 1,2-dichloroethane. The halogenating agent is used in an amount of at least 0.5 equivalents, preferably from 0.5 to 2 equivalents, per equivalent of the 2-(6-methylquinolin-2-yl)benzoic acid. The radical initiator is used in an amount of from 1 to 100 mg, preferably from 5 to 50 mg, per gram of the 2-(6-methylquinolin-2-yl)benzoic acid. It is preferable to effect the reaction under heating from 20 to 100°C for 0.1 to 10 hours, and preferably 0.5 to 2 hours. The 2-(6-methylquinolin-2-yl)benzoic acid may be produced by the publicly known method as described above.

After the completion of the reaction, the resulting reaction mixture is allowed to cool to room temperature. Then the solid thus precipitated is collected by filtration and washed with an appropriate organic solvent (for example, the one employed in the reaction) to thereby give the target compound. If necessary, the thus obtained product may be further purified.

When the secondary amine is a heterocyclic secondary amine represented by formula (2), then the target 6-aminomethyl-substituted quinolinebenzoic acid thus obtained is a compound represented by the following formula (4). When the secondary amine employed is a substituted 1H-imidazole represented by formula (3), then the thus obtained 6-aminomethyl-substituted quinolinebenzoic acid is a compound represented by the following formula (5). Of these, the preferred product is one represented by formula (4). The most desirable product is 2-{6-[(2-ethyl-5,7-dimethyl-3H-imidazo[4,5-b]pyridin-3-yl]quinolin-2-yl}benzoic acid, as shown in the following Examples.

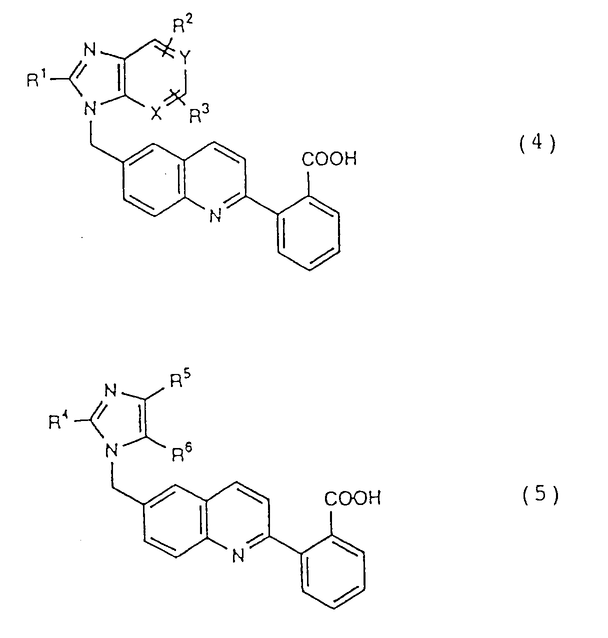

To further illustrate the present invention in greater detail, and not by way of limitation, the following Examples will be given.

### EXAMPLE 1 (Reference Example)

### Synthesis of 2-[6-(bromomethyl)quinolin-2-yl]benzoic acid:

To a mixture of a 1.0 g of 2-(6-methylquinolin-2-yl)benzoic acid with 50 ml of 1,2-dichloroethane were added 0.7 g of N-bromosuccinimide (NBS) and 20 mg of azobisisobutyronitrile (AIBN). The resulting mixture was heated under reflux for 1 hour and then allowed to stand at room temperature. The crystals thus precipitated were collected by filtration to thereby give 0.9 g of 2-[6-(bromomethyl)quinolin-2-yl]benzoic acid.

NMR (400 MHz, CDCl₃): δ 7.26 - 8.38 (m, 9H); 4.69 (s, 2H).

### EXAMPLE 2

### Synthesis of 2-{6-[(2-ethyl-5,7-dimethyl-3H-imidazo[4,5-b]pyridin-3-yl)methyl]quinolin-2-yl}benzoic acid:

2-Ethyl-5,7-dimethyl-1H-imidazo[4,5-b]pyridine (0.21 g) was dissolved in 6 ml of N,N-dimethylformamide and 0.12 g of sodium hydride was added thereto under ice-cooling. After stirring the mixture at room temperature for 1 hour, 0.5 g of the 2-[6-(bromomethyl)quinolin-2-yl]benzoic acid obtained in Example 1 was added thereto under ice-cooling. After stirring for 2 hours, the reaction mixture was poured into an aqueous solution of sodium chloride and washed with toluene. Next, the aqueous layer was neutralized with 3 N hydrochloric acid until the pH value reached 5. The precipitate thus formed was collected by filtration to thereby give 0.44 g of 2-{6-[(2-ethyl-5,7-dimethyl-3H-imidazo[4,5-b]pyridin-3-yl)methyl]quinolin-2-yl}benzoic acid.

NMR (400 MHz, CDCl₃): δ 7.58 - 8.26 (m, 8H); 7.43 (s, 1H); 6.93 (s, 1H); 5.66 (s, 2H); 2.78 (q, J = 7.5 Hz, 2H); 2.65 (s, 3H); 2.59 (s, 3H); 1.27 (t, J = 7.5 Hz, 3H).

### EXAMPLE 3

### Synthesis of 2-{6-((2-ethyl-5,7-dimethyl-3H-imidazo[4,5-b]pyridin-3-yl)methyl]quinolin-2-yl}benzoic acid:

To 15 ml of 2-propanol were added 0.3 g of 2-ethyl-5,7-dimethyl-1H-imidazo[4,5-b]pyridine and 0.3 g of the 2-[6-(bromomethyl)quinolin-2-yl]benzoic acid obtained in Example 1. After adding 3 ml of a 1 N aqueous solution of sodium hydroxide, the resulting mixture was stirred for 2 hours under heating to 55°C. Then the reaction mixture was poured into an aqueous solution of sodium chloride and washed with toluene. Next, the aqueous layer was neutralized with 3 N hydrochloric acid until the pH value reached 5. The precipitate thus formed was collected by filtration to thereby give 0.18 g of 2-{6-[(2-ethyl-5,7-dimethyl-3H-imidazo[4,5-b]pyridin-3-yl)methyl]quinolin-2-yl}benzoic acid.

### EXAMPLE 4

### Synthesis of 2-{6-[(2-ethyl-5,7-dimethyl-3H-imidazo[4,5-b]pyridin-3-yl)methyl]quinolin-2-yl}benzoic acid:

To 1 ml of t-butyl alcohol were added 22 mg of 2-ethyl-5,7-dimethyl-1H-imidazo[4,5-b]pyridine and 21.5 mg of the 2-[6-(bromomethyl)quinolin-2-yl]benzoic acid obtained in Example 1. After adding 40 mg of potassium t-butoxide, the resulting mixture was stirred for 30 minutes under heating to 85°C. Then the reaction mixture was concentrated, diluted with water and washed with toluene. Next, the aqueous layer was neutralized with 3 N hydrochloric acid until the pH value reached 5. The precipitate thus formed was collected by filtration to thereby give 18 mg of 2-{6-[(2-ethyl-5,7-dimethyl-3H-imidazo[4,5-b]pyridin-3-yl)methyl]quinolin-2-yl}benzoic acid.

### EXAMPLE 5

### Synthesis of 2-{6-[(2-ethyl-5,7-dimethyl-3H-imidazo[4,5-b]pyridin-3-yl)methyl]quinolin-2-yl}benzoic acid:

2-Ethyl-5,7-dimethyl-1H-imidazo[4,5-b]pyridine (0.25 g) was dissolved in 10 ml of N,N-dimethylformamide. After successively adding thereto 0.3 g of potassium t-butoxide and 0.5 g of the 2-[6-(bromomethyl)quinolin-2-yl]benzoic acid obtained in Example 1 under ice-cooling, the resulting mixture was stirred under ice-cooling for 2 hours. Then the reaction mixture was poured into an aqueous solution of sodium chloride and washed with toluene. Next, the aqueous layer was neutralized with 3 N hydrochloric acid until the pH value reached 5. The precipitate thus formed was collected by filtration to thereby give 0.11 g of 2-{6-[(2-ethyl-5,7-dimethyl-3H-imidazo[4,5-b]pyridin-3-yl)methyl]quinolin-2-yl}benzoic acid.

### EXAMPLE 6

### Synthesis of 2-{6-[(2-ethyl-5,7-dimethyl-3H-imidazo[4,5-b]pyridin-3-yl)methyl]quinolin-2-yl}benzoic acid:

2-Ethyl-5,7-dimethyl-1H-imidazo[4,5-b]pyridine (0.1 g) was dissolved in 5 ml of N-methyl-2-pyrrolidone. After successively adding thereto 0.15 g of potassium t-butoxide and 0.1 g of the 2-[6-(bromomethyl)quinolin-2-yl]benzoic acid obtained in Example 1 under ice-cooling, the resulting mixture was stirred under ice-cooling for 2 hours. Then the reaction mixture was poured into an aqueous solution of sodium chloride and washed with toluene. Next, the aqueous layer was neutralized with 3 N hydrochloric acid until the pH value reached 5. The precipitate thus formed was collected by filtration to thereby give 49 mg of 2-{6-[(2-ethyl-5,7-dimethyl-3H-imidazo[4,5-b]pyridin-3-yl)methyl]quinolin-2-yl}benzoic acid.

As discussed above, the present invention makes it possible to easily produce 2-{6-[(2-ethyl-5,7-dimethyl-3H-imidazo[4,5-b]pyridin-3-yl)methyl]quinolin-2-yl}benzoic acid, which is useful as an angiotensin II antagonist, in a short step.

While the invention has been described in detail and with reference to specific examples thereof, it will be apparent to one skilled in the art that various changes and modifications can be made therein without departing from the spirit and scope thereof.

## Claims

1. A process for producing a 6-aminomethyl-substituted quinolinebenzoic acid wherein an amine residue which is formed by eliminating the hydrogen atom bound to the nitrogen atom of a secondary amine is bound to the methyl group at the 6-position of the quinoline skeleton,
which comprises reacting a 2-{6-(halomethyl)quinolin-2-yl}benzoic acid represented by the following formula (1) with the secondary amine in the presence of a base: 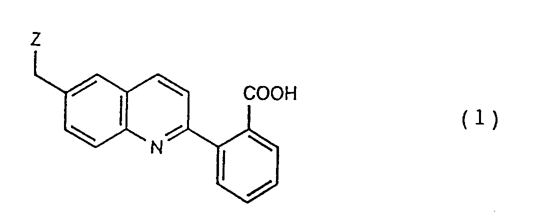 wherein Z represents a chlorine, bromine or iodine atom.

2. The process as claimed in claim 1, wherein said secondary amine is a heterocyclic compound having a ring containing a nitrogen atom to which a hydrogen atom is bound.

3. The process as claimed in claim 1, wherein said secondary amine is a substituted 3H-imidazo[4,5-b]pyridine or a substituted 1H-imidazole.

4. The process as claimed in claim 1, wherein said secondary amine is a 3H-imidazo[4,5-b]pyridine represented by the following formula (2): 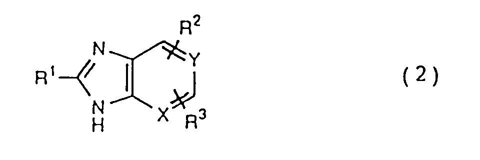 wherein R¹ represents a lower alkyl, halo lower alkyl, cyclo lower alkyl, lower alkenyl, lower alkoxy, alkoxy lower alkyl or lower alkylthio group;
R² and R³ each independently represents a hydrogen atom, a halogen atom, a lower alkyl, halo lower alkyl, cyclo lower alkyl, alkenyl or alkoxy group, -CₘF₂ₘ₊₁, -(CH₂)ₙR⁷, -(CH₂)ₚCOR⁸ or -(CH₂)_{q}NR⁹COR¹⁰; and
X and Y each independently represents =CH or =N,
in which R⁷ represents a hydroxyl or lower alkoxy group;
R⁸ and R¹⁰ each independently represents a hydrogen atom, a hydroxyl, lower alkyl or lower alkoxy group or -NR¹¹R¹², in which R¹¹ and R¹² each independently represents a hydrogen atom or a lower alkyl group;
R⁹ represents a hydrogen atom or a lower alkyl group;
m is an integer of from 1 to 6;
n is an integer of from 1 to 4;
p is an integer of from 0 to 4; and
q is an integer of from 1 to 4.

5. The process as claimed in claim 4, wherein R¹, R² and R³ each is an alkyl group having not more than 6 carbon atoms; X is =N; and Y is =CH.

6. The process as claimed in claim 1, wherein said secondary amine is a 3H-imidazo[4,5-b]pyridine represented by the following formula (2'): 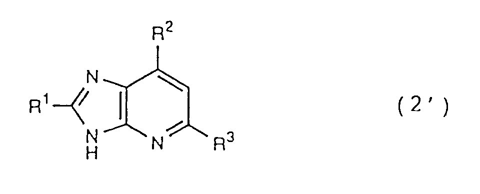 wherein R¹ is an alkyl or haloalkyl group having not more than 6 carbon atoms; and
R² and R³ each independently is a hydrogen atom or an alkyl group having not more than 6 carbon atoms.

7. The process as claimed in claim 1, wherein said secondary amine is 2-ethyl-5,7-dimethyl-3H-imidazo[4,5-b]pyridine, and said 6-aminomethyl-substituted quinolinebenzoic acid is 2-{6-[(2-ethyl-5,7-dimethyl-3H-imidazo[4,5-b]pyridin-3-yl)methyl]quinolin-2-yl}benzoic acid.

8. The process as claimed in claim 1, wherein said 2-{6-(halomethyl)quinolin-2-yl}benzoic acid is 2-{6-(bromomethyl)quinolin-2-yl}benzoic acid.

9. The process as claimed in claim 1, wherein said base is at least one selected from the group consisting of alkali metal hydrides, alkali metal hydroxides, alkali metal alkoxides and alkali metal carbonates.

10. The process as claimed in claim 1, wherein said reaction between a 2-{6-(halomethyl)quinolin-2-yl}benzoic acid and a secondary amine is performed in an aprotic solvent at a temperature of from 0°C to the reflux temperature of the solvent.
